# EUROPEAN PATENT SPECIFICATION

(11) **EP 3 884 814 B1**
(45) Date of publication and mention of the grant of the patent: **21.05.2025**
(21) Application number: 19895562.7
(22) Date of filing: 15.10.2019
(51) Int. Cl.: A47G 9/10, A61F 5/56, A61B 5/00, A47G 9/00

(54) **EXTERNAL FORCE DETECTING SYSTEM AND METHOD FOR DRIVING EXTERNAL FORCE DETECTING SYSTEM**
SYSTEM ZUR ERFASSUNG EXTERNER KRÄFTE UND VERFAHREN ZUR ANSTEUERUNG EINES SYSTEMS ZUR ERFASSUNG EXTERNER KRÄFTE
SYSTÈME DE DÉTECTION DE FORCE EXTERNE ET PROCÉDÉ DE PILOTAGE DE SYSTÈME DE DÉTECTION DE FORCE EXTERNE

(30) Priority: 13.12.2018 KR 20180160801; 09.04.2019 KR 20190041223
(43) Date of publication of application: 29.09.2021
(73) Proprietor: Tenminds Co., Ltd., Seoul 06120 (KR)
(72) Inventor: JANG, Seung Woong, Seoul 06784 (KR); GAO, Lanzhou, Xiamen, Fujian 361000 (CN)
(74) Representative: Isarpatent
(86) International application number: PCT/KR2019/013469
(87) International publication number: WO 2020/122394

(56) References cited:
- CN-A- 108 969 175
- JP-U- 3 071 127
- KR-A- 20180 070 331
- KR-A- 20180 070 331
- KR-A- 20180 126 821
- KR-A- 20180 130 928
- KR-B1- 101 643 545

## Description

### TECHNICAL FIELD

The present invention relates to an external force detecting system and a method for driving the external force detecting system.

### BACKGROUND ART

Recently, the Internet of Things (IoT), which refers to a technology or environment in which a sensor is attached to an object to exchange data through the Internet in real time, is attracting attention.

There are no specific restrictions on the types of things to which the IoT is applied, and one of such things is an intelligent pillow or a smart pad which alleviates the symptoms of a user's sleep disorder and help them sleep well.

Korean Patent Application Publication No. 10-2008-0075263 discloses a device for adjusting the height of a pillow using an air cell, in which the air cell is installed inside the pillow, and a plurality of body pressure measurement sensors are attached to the outer surface of the pillow to automatically adjust the air pressure of the air cell according to the shape of the head and neck and the pressing force thereof.

Korean Patent No. 10-0788715 discloses a pillow whose height is automatically adjusted, which includes a sensing unit installed on the upper surface of the outer skin of the pillow body to detect the user's sleeping posture and a touch switch that generates an electrical signal when touched by the conductive human skin.

However, if the body pressure sensor or sensing unit is installed on the outer skin of the pillow, it may cause discomfort from pressing the head and face, which may rather interfere with the user's sound sleep. Further, the body pressure sensor is not easy to purchase on the market and, although DIYed, it may be very pricey due to its high product costs.

Korean Patent Application Publication No. 10-2010-0124613 discloses a pillow for preventing snoring, in which a signal detection unit is provided on each of the left and right ends of the pillow body to detect a snoring sound, and more air is injected to the air bag on the side where the stronger sound is detected to move the user's head and hence prevent snorting.

In practice, however, it is not easy to detect the sound intensity of snoring distinctly for the left and right, nor is it easy to move the user's head with only the left and right air pockets when the user snores while lying face up on the pillow.

Korean Patent No. 10-0758780 discloses a pillow with a built-in speaker. However, as the speaker is built into the pillow itself, it may press on the user's head, causing discomfort while lying on the pillow.

As described above, the pillow or pad according to the prior art can alleviate the symptoms of sleep disorder and automatically adjust the height. To that end, the pillow or pad should be able to first detect the position of the user's head or the position to which an external force is applied.

In order to realize such position detection, a plurality of sensing means or electronic components are arranged on the pillow itself, but this may result in a malfunction or damage and inaccurate detection. In addition, a sensitive user may feel uncomfortable due to foreign objects, noise, and vibration, and the user's body may be exposed to electromagnetic waves.

CN 108 969 175 A provides a kind of head position detection method and easy pillows, according to the preamble of claim 1.

### DETAILED DESCRIPTION OF THE INVENTION

### TECHNICAL OBJECTS

Some embodiments of the present invention aim to provide an external force detecting system and method for driving the external force detecting system, which has the portion where the user's head is placed configured as simple as possible to eliminate the risk of damage or malfunction and may be able to more accurately detect the position where an external force is applied without misdetermination.

Further, some embodiments of the present invention aim to an external force detecting system and method for driving the external force detecting system, which may alleviate snoring or teething during sleep or sleep apnea.

Further, some embodiments of the present invention aim to provide an external force detecting system and method for driving the external force detecting system, which may be operated in a customized manner considering the fact that a different external force per user is applied.

However, the objects of the embodiments are not limited thereto, and other objects may also be present.

### MEANS TO ACHIEVE THE OBJECTS

To achieve the foregoing objectives, as a technical means, according to the present invention, an external force detecting system of a pillow, as defined by independent claim 1, comprises a body including a plurality of same air bags, a driver including a motor pump and a processor, the motor pump injecting air to expand the plurality of air bags or discharging air to contract the air bags, the processor controlling operations of the motor pump and detecting an air bag pressurized by an external force applied to the body, a plurality of air passages being identical in number to the plurality of air bags, the plurality of air passages having first ends individually connected to the air bags and second ends connected with the driver, and an air pressure sensing unit disposed between the first ends and the motor pump to measure air pressures of the air bags into which the same amount of air has been injected.

The processor obtains a maximum air pressure and a minimum air pressure among the measured air pressures and compares a difference between the maximum air pressure and the minimum air pressure with a threshold to thereby determine whether the detected air bag is pressurized by a user.

In some embodiments, the processor receives the user's physical information, obtains the threshold according to a weight of the user's head based on the physical information, and compares the difference with the obtained threshold to thereby determine whether the detected air bag is pressurized by the user's head.

In some embodiments, the driver further includes a sound receiver receiving a sound from surroundings, and the processor determines whether the user is in any abnormal state of snoring during sleep, teething during sleep, or sleep apnea, based on a result of analysis of the received sound.

When the user is in the abnormal state, the processor controls to allow a larger amount of air than the same amount to be injected into the detected air bag.

In some embodiments, when the driver is implemented as a casing including a predetermined space, a connector including a plurality of through holes is formed on a first surface of the casing. The second ends are coupled from an outside of the casing to the through holes in a one-to-one correspondence. A plurality of air pathways connected from an inside of the casing to the motor pump are coupled to the second ends in a one-to-one correspondence. The air pressure sensing unit is disposed in the casing.

In some embodiments, the driver further includes a main air pathway connected with the motor pump, a plurality of sub air pathways branched from the main air pathway and connected with the second ends in a one-to-one correspondence, and a valve group installed between the motor pump and the through holes to block or open an air flow. The processor controls operations of individual valves included in the valve group to sequentially inject the same amount of air into the air bags.

The air pressure sensing unit includes a plurality of air pressure sensors individually disposed on the sub air pathways. The valve group includes a plurality of valves disposed between the air pressure sensors and the main air pathway.

The air pressure sensing unit includes a main air pressure sensor disposed on the main air pathway. The valve group includes a plurality of valves individually disposed on the sub air pathways and a main valve disposed on the main air pathway between the main air pressure sensor and the motor pump.

In some embodiments, the body is formed of memory foam. A predetermined pattern is engraved in a surface of the body.

In some embodiments, a power supply connecting terminal separate from the through holes is further included in the connector. When power is applied from the power supply connecting terminal, the driver automatically initiates a predetermined series of driving processes.

To achieve the foregoing objectives, according to the present invention, a method for driving an external force detecting system of a pillow, as defined by independent claim 11, comprises controlling, by a driver, to inject the same amount of air into a plurality of same air bags, which are in an initial state in a body spaced apart from the driver, via air passages individually connected with the air bags, measuring, by an air pressure sensing unit spaced apart from the body, air pressures of air bags into which the same amount of air has been injected, detecting, by the driver, an air bag pressurized by an external force applied to the body by comparing the measured air pressures, obtaining, by the driver, a maximum air pressure and a minimum air pressure among the measured air pressures, and determining, by the driver, whether the detected air bag is pressurized by a user by comparing a difference between the maximum air pressure and the minimum air pressure and a threshold.

In some embodiments, the method further comprises receiving, by the driver, the user's physical information and obtaining, by the driver, the threshold according to a weight of the user's head based on the physical information, wherein said determining includes comparing the difference with the obtained threshold to thereby determine whether the detected air bag is pressurized by the user's head.

In some embodiments, the method further comprises receiving, by a sound receiver disposed in the driver, a sound from surroundings, determining, by the driver, whether the user is in any abnormal state of snoring during sleep, teething during sleep, or sleep apnea, based on a result of analysis of the received sound, and upon determining that the detected air bag is pressurized by the user and that the user is in the abnormal state, controlling, by the driver, to allow a larger amount of air than the same amount to be injected into the detected air bag.

In some embodiments, the driver further includes a motor pump, a main air pathway connected with the motor pump, a plurality of sub air pathways branched from the main air pathway and connected with the air passages in a one-to-one correspondence, and a valve group installed between the motor pump and the air passages to block or open an air flow.

Said controlling includes controlling operations of the motor pump and individual valves included in the valve group to sequentially inject the same amount of air into the plurality of same air bags which are in the initial state.

In some embodiments, said controlling, said measuring, said detecting, said obtaining, and said determining are performed automatically as a predetermined series of driving processes when power is applied to the driver.

### EFFECTS OF THE INVENTION

The external force detecting system and the method for driving the same proposed according to the present invention are expected to have the following effects.

The body of the external force detecting system includes no sensing means or electronic component and be thus freed from any concern about damage or malfunction or may significantly reduce risk of electromagnetic waves.

Further, the body of the external force detecting system has a very simple structure. Thus, even when the user's head is positioned on the body, the user may be avoided from any disturbance while sleeping.

Further, the driver of the external force detecting system may inject a minimum amount of air for air pressure measurement, thus allowing for accurate detection of the position of the user's head without disturbing the sleeping user.

Further, the air pressure sensing unit of the external force detecting system need not have high sensitivity, and this may provide an advantage in light of costs, as compared with methods adopting other sensing means.

Further, according to some embodiments, it is possible to quickly and precisely detect the position where an external force is applied to the body, by the simplified method which measures air pressure and to mitigate or remove the abnormal state of the sleeping user using the detected position information.

Further, although the user's head and other body portion (e.g., the user's hand) both are placed in different positions of the body of the system, the position of the user's head may be accurately detected using the maximum air pressure and minimum air pressure.

Further, even when a thing other than the user's head is placed on the body of the system, the system and method may compare the difference between the maximum air pressure and minimum air pressure and a threshold and use the result of comparison, thereby preventing the other thing from being mistaken for the user's head and resultant malfunctions.

Further, in some embodiments, the threshold according to the weight of the user's head may be calculated, and the calculated threshold may be put to use. The threshold may be set to differ per user so that the optimal operation is rendered possible for the corresponding user.

The history information about the operation of the system may be provided to the user's communication device via a dedicated application to enable user-customized analysis reporting and mitigation of the abnormal state while the user is sleeping. Thus, the user's satisfaction may be increased.

Further, no separate power on/off buttons are provided so that applying power, itself, may mean a state in which driving has started or a state in which a start signal is awaited. Thus, in contrast to the typical or conventional process for driving a wired electronic product, which requires two steps (of connecting the power line and pressing the power-on button), the driving process may be simplified into one step, increasing the user convenience.

### BRIEF DESCRIPTION OF THE DRAWINGS

Fig. 1 is a view schematically illustrating a configuration of an external force detecting system according to an embodiment of the present invention;
Fig. 2 is a block diagram illustrating a processor according to an embodiment of the present invention;
Fig. 3 is a view illustrating detailed components of a driving unit and a correction relationship therebetween, according to an embodiment of the present invention;
Fig. 4 is a flowchart illustrating a method for driving an external force detecting system according to an embodiment of the present invention;
Fig. 5 is a flowchart illustrating an external force detecting method by an external force detecting system according to an embodiment of the present invention;
Fig. 6 is a view illustrating an example of influence on a user as an external force detecting system is driven according to an embodiment of the present invention;
Fig. 7 is a view illustrating detailed components of a driving unit and a correction relationship therebetween, according to another embodiment of the present invention;
Fig. 8 is a flowchart illustrating a method for driving an external force detecting system according to another embodiment of the present invention; and
Fig. 9 is a view illustrating example main components of an external force detecting system according to an embodiment of the present invention.

### MODE TO PRACTICE THE INVENTION

Hereinafter, embodiments of the present invention are described below with reference to the accompanying drawings for one of ordinary skill in the art to easily practice the present invention. However, the present invention may be implemented in other various forms and is not limited to the embodiments set forth herein. For clarity, components or parts irrelevant to the present invention are omitted from the drawings or the detailed description.

In embodiments of the present invention, when an element is "connected" with another element, the element may be "directly connected" with the other element, or the element may be "electrically connected" with the other element via an intervening element. When an element "includes" another element, the element may further include the other element, rather excluding the other element, unless particularly stated otherwise.

Hereinafter, embodiments of the disclosure are described in detail with reference to the accompanying drawings.

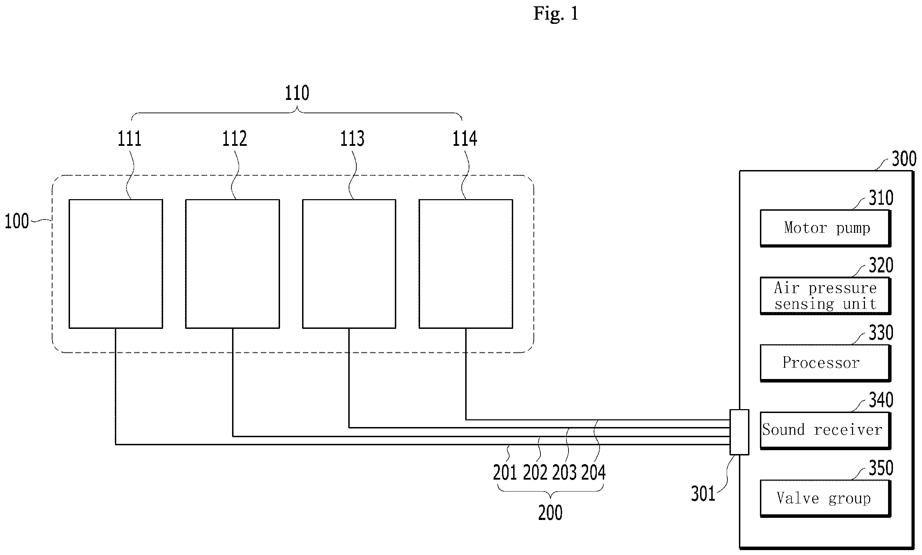

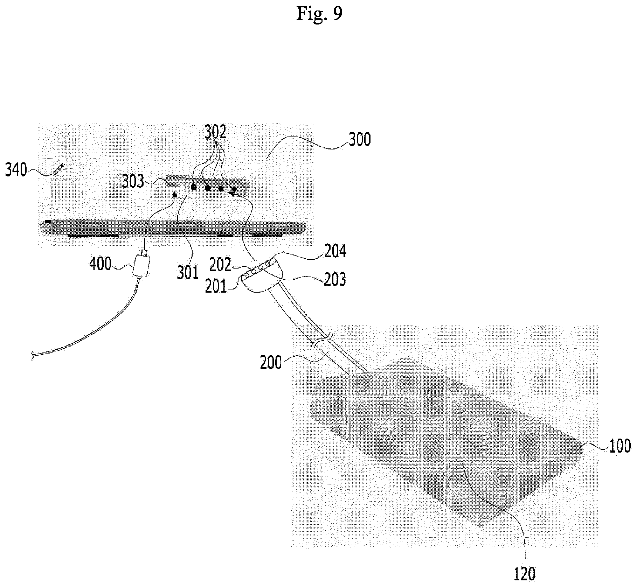

Fig. 1 is a view schematically illustrating an external force detecting system according to an embodiment of the present invention. Fig. 9 is a view illustrating example main components of an external force detecting system according to an embodiment of the present invention.

An external force detecting system includes a body 100 and a driver 300 connected with the body 100 via an air passage 200.

The body 100 includes a plurality of same air bags 110 which may be expanded or contracted.

Here, "same air bags 100" means that the air bags 100 have substantially the same size, shape, material, expansion coefficient, maximum air capacity, or characteristic. Although Fig. 1 illustrates that the body 100 includes four rectangular air bags spaced apart from one another at a predetermined interval in the left and right directions, the number, shape, interval, and arrangement of the air bags are not limited to those shown.

Initially, the air bags 110 may remain flat with no air injected. If air has been injected into any air bag, a process for returning the air bag to the initial state, that is, a state in which there is substantially no air injection, may be performed.

The body 100 may be configured to be placed under the lower head of the user to receive the user's head. The body 100 may be implemented as, e.g., a pillow, pad, or cushion. Thus, at least the user's head may pressurize a specific spot of the body 100 in an upper or lower direction while the user is sleeping.

As shown in Fig. 9, the body 100 may be formed of memory foam or elastic material to stably support the user's head. The surface of the body portion 100 may be engraved or grooved to have a predetermined pattern, such as a grid pattern. The engraved portion 120 may allow the body 100 to naturally transform and deliver more comfort to the user while the user's head is placed on top of the body 100.

The body 100 may include no sensing means or electronic component and be thus freed from any concern about damage or malfunction or may significantly reduce risk of electromagnetic waves. As the body 100 is formed with a very simplified structure as described above, the user may be free from discomfort or sleep disturbance that would otherwise arise from its internal configuration.

The number of air passages 200 may be the same as the number of air bags 110. The respective first ends of the air passages 200 may be individually connected to the air bags of the body 100 (e.g., in a one-to-one correspondence), and the respective second ends of the air passages 200 may be connected to through holes 302 of the driver 300.

To be prevented from being twisted or tangled, the air passages 201, 202, 203, and 204 may be covered and fastened by an integrated tube or fastener as shown in Fig. 9. The second ends of the air passages 200 may be implemented with male-and-female coupling adapters.

Throughout the specification, the air bags are denoted a first air bag 111, a second air bag 112, a third air bag 113, and a fourth air bag 114 from the left to right. The first air passage 201, the second air passage 202, the third air passage 203, and the fourth air passage 204, respectively, are connected to the first air bag 111, the second air bag 112, the third air bag 113, and the fourth air bag 114 in a one-to-one correspondence manner.

The driver 300 is a component physically spaced apart from the body 100 and is connected to the body 100 via each air passage 200. Preferably, the driver 300 is spaced apart from the user's body at a predetermined distance or interval to reduce electromagnetic waves or noise.

The driver 300 includes a connector 301 which have as many through holes 302 as the number of air bags 110 in one side surface thereof.

Specifically, the driver 300 includes a motor pump 310, an air pressure sensing unit 320, a processor 330, a sound receiver 340, and a valve group 350. In other words, the driver 300 includes sensing means or electronic components unlike the body 100.

As shown in Fig. 9, the driver 300 may be implemented as a box-like casing with a predetermined space therein.

A connector 301 including a plurality of through holes 302 and a power supply connecting terminal 303 separated from the plurality of through holes 302 may be formed on one surface of the casing. A space and hole may be formed in the opposite surface of the casing to receive the sound receiver 340.

The respective second ends of the air passages 200 may be individually coupled from the outside of the casing to the plurality of through holes 302 in a one-to-one correspondence manner and be coupled with a plurality of air pathways connected to the motor pump 310 from the inside of the casing, in a one-to-one correspondence manner.

The motor pump 310 may inject air through the second ends of the air passages 200 into the air bags 110 to expand the air bags 110 or may discharge the air from the air bags 110 to contract the air bags 110.

The air pressure sensing unit 320 may be disposed between the motor pump 310 and the respective ends of the air passages 200 to measure the air pressure of the air bags 110 into which the same amount of air has been injected.

The air pressure sensing unit 320 may be disposed in a position other than in the body 100 (e.g., the air pressure sensing unit 320 may be placed in a specific position on the air passages 200 or, if the driver 300 is implemented as a casing, the air pressure sensing unit 320 may be positioned inside the casing) and may control to allow the same amount of air to be sequentially injected into the air bags 100.

Here, the "same amount of air" may be typically determined in a range from 0.5% to 15% of the maximum air capacity. Where the same amount of air is less than 0.5% of the maximum air capacity, the accuracy of air pressure measurement may be about 90%. Where the same amount of air exceeds 15% of the maximum air capacity, the user may feel expansion or contraction of the air bag and thus discomfort while air is injected or discharged to measure the air pressure.

Thus, it is preferable that the same amount of air is determined in a range from 0.5% to 2% of the maximum air capacity. As an example, a test performed with air whose amount is 1% of the maximum air capacity injected into the air bags revealed that the accuracy of air pressure measurement and the time taken to expand one air bag were 99.9% and 0.7 seconds, respectively, and, during the course, the user felt no change in the air bags. As such, the same amount of air may be determined considering the accuracy (99.8% or more) of air pressure measurement, the time taken for the air pressure measurement process (within a few seconds), or whether it is sensed by the user.

Before the air pressure sensing unit 320 measures the air pressure, the same amount of air may be injected into each empty air bag 110, and such air injection process may be sequentially performed. For example, the same amount of air may be sequentially injected into the air bags 110 in the order of the first air bag 111, the third air bag 113, the fourth air bag 114, and the second air bag 112.

According to an embodiment, the air pressure sensing unit 320 may include a single air pressure sensor (which may be denoted a main air pressure sensor) and, in such a case, the air pressure measurement process may be sequentially performed like the air injection process. For example, air pressure measurement for the air bags 110 may be sequentially performed in the order of the first air bag 111, the third air bag 113, the fourth air bag 114, and the second air bag 112. After the air pressure measurement process is done, the air may be discharged out of each air bag 110 by the motor pump 310.

According to another embodiment, the air pressure sensing unit 320 may include a plurality of air pressure sensors in which case the air pressure measurement process may be simultaneously performed. For example, the air pressure of the first air bag 111 may be measured by a first air pressure sensor, and the air pressure of the second air bag 112 may be measured by a second air pressure sensor. After the air pressure measurement process is done, the air may be discharged out of each air bag 110 by the motor pump 310.

One or more processors 330 control the operations of the motor pump 310 and the other components of the driver 300. The processor 330 may be a central processing unit (CPU), a microprocessor unit (MPU), a micro-controller unit (MCU), or any kind or type of processor known in the art.

The processor 330 may perform computation on at least one application or program to execute the driving method according to some embodiments of the present invention.

The processor 330 may compare the air pressures measured by the air pressure sensing unit 320 and detect at least one air bag pressurized by the external force applied to the body 100.

The external force may mean a force exerted in the upper and lower directions.

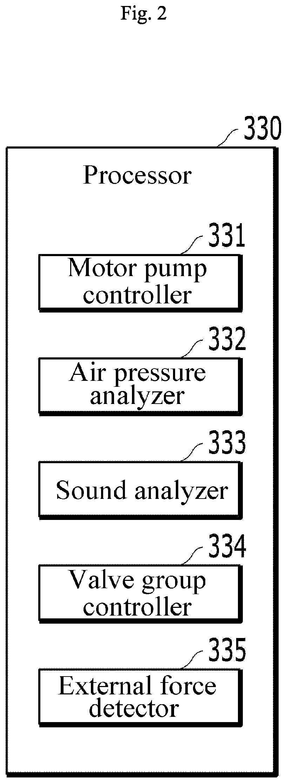

The processor 330 is described below in greater detail with reference to Fig. 2.

The sound receiver 340 may receive the sound generated from the surroundings, generate an electrical signal corresponding to the received sound, and transmit the electrical signal to the processor 330.

The sound receiver 340 may receive the sound generated from the sleeping user, and the processor 330 may analyze the received sound and use the result of analysis to grasp the user's current state or condition.

The sound receiver 340 may be implemented as, e.g., a microphone.

The valve group 350 may be installed between the motor pump 310 and the second ends of the air passages 200 or the through holes of the connector 301 and block or open the air flow according to control signals from the processor 330.

As described above, the driver 300 may have the connector 301, and the connector 301 may further include the power supply connecting terminal 303 separately from the through holes 302.

If the power supply connecting terminal 303 is connected with a power line 400 so that power is applied from the power supply connecting terminal 303, the driver 300 may automatically initiate and perform a series of driving processes, such as reception of sound, grasping the user's state or condition, sequential injection of the same amount of air, air pressure measurement, and external force detection, in a predetermined order even without the user's separate action or manipulation. The driving process may be periodically repeated.

The driver 300 may be configured with no separate power on/off button. As such, as no power on/off button is provided, the connection between the power supply connecting terminal 303 and the power line 400, itself, may mean a state in which driving has started or a state in which a start signal (e.g., reception of a sound) is awaited. Thus, in contrast to the typical or conventional process for driving a wired electronic product, which requires two steps (of connecting the power line and pressing the power-on button), the driving process may be simplified into one step, increasing the user convenience and satisfaction.

Additionally, the driver 300 may further include a memory (not shown) for storing various data, commands, and/or information, and a network interface (not shown).

The memory may be, e.g., an RAM, SRAM, DRAM, PSRAM, SDPARM, DDR SDRAM, or any other memories known in the art to which the present invention pertains.

The network interface may be connected with the user's communication device, server, or database via a network so as to perform communication.

The user's communication device may be implemented as a computer or a portable terminal. The computer may include, e.g., a WEB browser-equipped personal computer (PC), desktop computer, laptop computer, tablet PC, slate PC, or vehicle navigation device. Examples of the portable terminal may include portable and mobile wireless communication devices, e.g., a Personal Communication System (PCS), Global System for Mobile communications (GSM), Personal Digital Cellular (PDC), Personal Handyphone System (PHS), Personal Digital Assistant (PDA), International Mobile Telecommunication (IMT)-2000, Code Division Multiple Access (CDMA)-2000, W-Code Division Multiple Access (W-CDMA), Wireless Broadband Internet (WiBro) terminal, a smartphone, or any other various types of handheld wireless communication devices.

The network interface may be implemented as a communication module known in the art to which the present invention pertains, so as to support various communication schemes or standards. For example, the network interface may be implemented as a wired/wireless communication module, a network card, or an infrared (IR) communication module.

Wi-Fi, Wideband CDMA (WCDMA), High Speed Downlink Packet Access (HSDPA), High Speed Uplink Packet Access (HSUPA), High Speed Packet Access (HSPA), and World Interoperability for Microwave Access (WIMAX). , Mobile WiMAX, WiBro, 3GPP (3rd Generation Partnership Project), LTE (Long Term Evolution), LTE-A (Long Term Evolution-Advanced), Bluetooth, infrared communication (IrDA, infrared data association), NFC (Near Field Communication), Zigbee, LAN (Local Area Network), Wireless LAN (Wireless Local Area Network), WAN (Wide Area Network), and PAN (Personal Area Network) technologies may be applied to the network interface.

Where the network interface is connected to the Internet to provide services, it may follow the TCP/IP which is the standard protocol for information transmission on the Internet. In other words, the network interface may mean a global open computer network structure that provides HTTP (Hyper Text Transfer Protocol), Telnet, FTP (File Transfer Protocol), DNS (Domain Name System), SMTP (Simple Mail Transfer Protocol), SNMP (Simple Network Management Protocol), NFS (Network File Service), or NIS (Network Information Service).

As such, the driver 300 of the external force detecting system may perform communication with the user's communication device, and the user may receive an analysis report for abnormal conditions during sleep (e.g., snoring during sleep, teething during sleep, sleep apnea) via a dedicated application installed on the communication device and history information for the operation of the external force detecting system to improve the abnormal conditions. Further, the user may change the driving conditions or setting values of the driver 300 to be user-customizable via the dedicated application and may perform a test as to whether the air bag corresponding to the position pressurized as an external force is applied to any position of the body 100 is normally expanded.

Fig. 2 is a block diagram illustrating a processor according to an embodiment of the present invention.

Although Fig. 2 illustrates that the detailed components of the processor are separate components from one another, some or all of the components may be integrated into a single component. The detailed components may be implemented as different modules depending on their functions or may be implemented in such a manner that their respective coded programs are performed by a processor.

The processor 330 may include a motor pump controller 331, an air pressure analyzer 332, a sound analyzer 333, a valve group controller 334, and an external force detector 335.

The motor pump controller 331 may generate control signals for controlling the operation of the motor pump 310 to inject air into each air bag 110 or to discharge the air out of the air bag 110 and the motor pump controller 331 may interwork with the valve group controller 334.

The air pressure analyzer 332 may obtain the maximum air pressure and minimum air pressure among the measured air pressures and compare the difference between the maximum air pressure and the minimum air pressure with a threshold to determine whether the detected air bag is pressurized by the user.

Where the difference between the maximum air pressure and the minimum air pressure is equal to or larger than the threshold, the air pressure analyzer 332 may determine that the detected air bag is pressurized by the user (particularly, by the user's head) and that the user's head is positioned on the detected air bag. In such a case, the external force detector 335 may detect that an external force has been generated on the air bag, for which the maximum air pressure has been measured, of the body 100, by the user's head.

Where the difference between the maximum air pressure and the minimum air pressure is smaller than the threshold, the air pressure analyzer 332 may determine that the detected air bag is pressurized by the user's body portion than the user's head, or a thing (e.g., the user's mobile phone, tablet PC, or book) other than the user's head. In such a case, the external force detector 335 may detect that an external force has been generated on the air bag, for which the maximum air pressure has been measured, of the body 100, by the user's body portion other than the user's head or a thing other than the user's head.

Setting the threshold as such may minimize the processor 330's mistaking another item or user's hand for the user's head although the other item or user's hand is placed on the body 100. Further, because the maximum air pressure and the minimum air pressure are used, although both the user's head and hand are placed in different positions of the body 100, the processor 330 may rapidly and accurately detect the position of the user's head.

The above-described threshold may be adjusted by the user and, according to an embodiment, the threshold may be calculated according to the weight of the user's head.

The air pressure analyzer 332 may receive physical information about the user and calculate the threshold according to the weight of the user's head, based on the physical information.

For example, the user may input physical information, such as her gender, age, and weight, via a dedicated application, and the air pressure analyzer 332 may obtain average head weight data corresponding to the physical information using statistical data regarding head weights.

In general, a person's head weight ranges from 4.5kg to 6.5kg. Thus, the obtained head weight data may be divided into the case of being less than 4.5kg, the case of being not less than 4.5kg and less than 5.5kg, the case of being not less than 5.5kg and less than 6.5kg, and the case of being 6.5kg and more, and for each case, a different threshold may be calculated.

By so doing, the air pressure analyzer 332 may more accurately determine whether the detected air bag is pressurized by the user's head by comparing the difference with the calculated threshold, and the threshold may be set in a user customized manner.

The sound analyzer 333 may determine whether the user is in any one abnormal state of snoring during sleep, teething during sleep, or sleep apnea based on the result of analysis of the sound received from the sound receiver 340.

In analyzing the sound, noise may be filtered out, and user's sound information may be extracted. The extracted user sound information may be compared with sound pattern information conventionally known for the abnormal state. A reference decibel value may be set so that the user sound information may be extracted when the maximum value of the sound signal is above a predetermined decibel.

Where the user is in the abnormal state, the process of measuring the air pressure of each air bag 110 may be performed.

The valve group controller 334 may generate a control signal for controlling the operation of each valve included in the valve group 350, thereby blocking or opening the air flow.

The valve group controller 334, along with the motor pump controller 331, may control the operation of the individual valves and the motor pump 310 so that the same amount of air is sequentially or simultaneously injected into the air bags 110.

The valve group controller 334, along with the motor pump controller 331, may control the operation of the individual valves and the motor pump 310 so that the injected air is sequentially or simultaneously discharged from the air bags 110.

The external force detector 335 may detect at least one air bag pressurized by the external force, in the upper and lower directions, applied to the body 100, using each measured air pressure or the result of analysis by the air pressure analyzer 332.

Where the user is in the abnormal state, the motor pump controller 331 may control to allow more air than the same amount of air to be injected into the air bag detected by the external force detector 335.

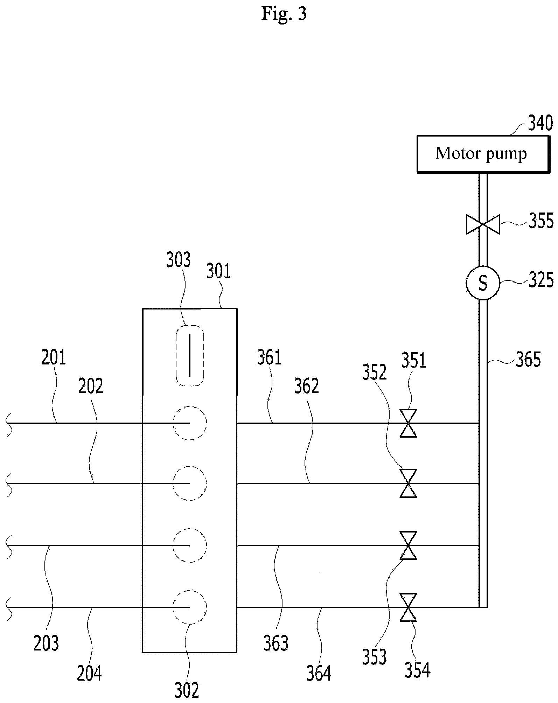

Fig. 3 is a view illustrating detailed components of a driving unit and a correction relationship therebetween, according to an embodiment of the present invention.

According to an embodiment, a connector 301 may be formed on one surface of the driver, and the connector 301 may include a plurality of through holes 302 as shown in Fig. 3.

According to an embodiment, the driver may further include a main air pathway 365 connected with the motor pump 310, a plurality of sub air pathways 361, 362, 363, and 364 branched from the main air pathway 365 and respectively connected with the second ends of the air passages 201, 202, 203, and 204 in a one-to-one correspondence manner, and a valve group installed between the motor pump 310 and the through holes 302 to block or open the air flow.

In other words, the first air passage 201 is connected with the first sub air pathway 361 via the first through hole, the second air passage 202 is connected with the second sub air pathway 362 via the second through hole, the third air passage 203 is connected with the third sub air pathway 363 via the third through hole, and the fourth air passage 204 is connected with the fourth sub air pathway 364 via the fourth through hole.

According to an embodiment, the air pressure sensing unit of the driver may include a main air pressure sensor 325 disposed on the main air pathway 365. The valve group may include a plurality of valves 351, 352, 353, and 354 disposed on the sub air pathways 361, 362, 363, and 364 and a main valve 355 disposed on the main air pathway 365 between the main air pressure sensor 325 and the motor pump 310.

The processor may control the operation of the individual valves of the valve group so that the same amount of air is sequentially injected into the air bags 110.

First, the processor may turn on the first valve 351 and the main valve 355 and turn off the other valves 352, 353, and 354 and then inject as much air as V into the first air bag 111. After injecting as much air as V, the processor turns off the main valve 355 and measures the air pressure of the first air bag 111. After measuring the air pressure of the first air bag 111, the processor discharges the air out of the first air bag 111.

Next, the processor may turn on the second valve 352 and the main valve 355 and turn off the other valves 351, 353, and 354 and then inject as much air as V, which is the same amount of air injected into the first air bag 111, into the second air bag 112. After injecting as much air as V, the processor turns off the main valve 355 and measures the air pressure of the second air bag 112. After measuring the air pressure of the second air bag 112, the processor discharges the air out of the second air bag 112.

Next, the processor measures the air pressure of the third air bag 113 and the fourth air bag 114 in the same manner as described above.

According to an embodiment, the driver controls the operation of the motor pump 310 and the individual valves of the valve group as described above and may thus measure the air pressure of each air bag 110 under the same condition using the main air pressure sensor 325.

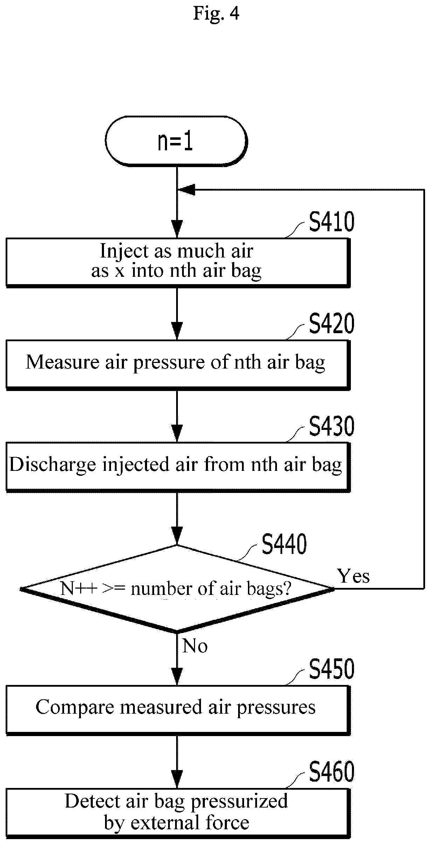

Fig. 4 is a flowchart illustrating a method for driving an external force detecting system according to an embodiment of the present invention. The driving method is described with reference to the detailed configurations of the drivers in the external force detecting system of Fig. 1 and the external force detecting system of Fig. 3.

As described above, the driver 300, which is spaced apart from, and separately configured from, the body 100, includes the air pressure sensing unit 320, the processor 330, and the motor pump 310 which injects or discharges air via the plurality of air passages 200 individually connected with the air bags 110.

First, the driver 300 or processor 330 controls to inject the same amount of air into the plurality of same air bags 110, which stays in the initial state in the body 100 spaced apart from the driver 300. In other words, before the air is injected, the air bags 110 are in substantially the same state.

If a certain air bag is in the state in which air has been injected thereinto, unlike the other air bags, a separate operation for discharging the air out of the certain air bag may be performed.

Next, the air pressure sensing unit 320 (or 325) is spaced apart from the body 100 and measures the air pressure of each of the air bags 110 into which the same amount of air has been injected.

Specifically, in step S410, by the operation of the processor 330, the motor pump 310, and the individual valves 351 to 355 of the valve group 350, as much air as x may be injected into the nth air bag. In step S420, the air pressure sensing unit 320 (or 325) may measure the air pressure of the nth air bag. In step S430, by the operation of the processor 330, the motor pump 310, and the individual valves 351 to 355 of the valve group 350, the air injected into the nth air bag may be discharged.

Here, n increases by one from one, and steps S410 to S430 are performed repeatedly as many times as the number of the air bags.

More specifically, the driver 300 may further include a main air pathway 365 connected with the motor pump 310, a plurality of sub air pathways 361 to 364 branched from the main air pathway 365 and connected with the air passages 200 in a one-to-one correspondence manner, and a valve group 350 installed between the motor pump 310 and the air passages 200 to block or open the air flow.

In the process of sequentially injecting air into the air bags, the operation of the motor pump 310 and the individual valves 351 to 355 included in the valve group 350 may be controlled to sequentially inject as much air as x, which is the same amount of air, into the air bags which are in the initial state.

In step S440, if n is equal to or smaller than the number of air bags, n is increased by one, and step S410 is performed. If n is larger than the number of air bags, step S450 is performed.

In step S450, the driver 300 or the processor 330 compares the measured air pressures. In step S460, the driver 300 or processor 330 detects the air bag pressurized by the external force applied to the body 100.

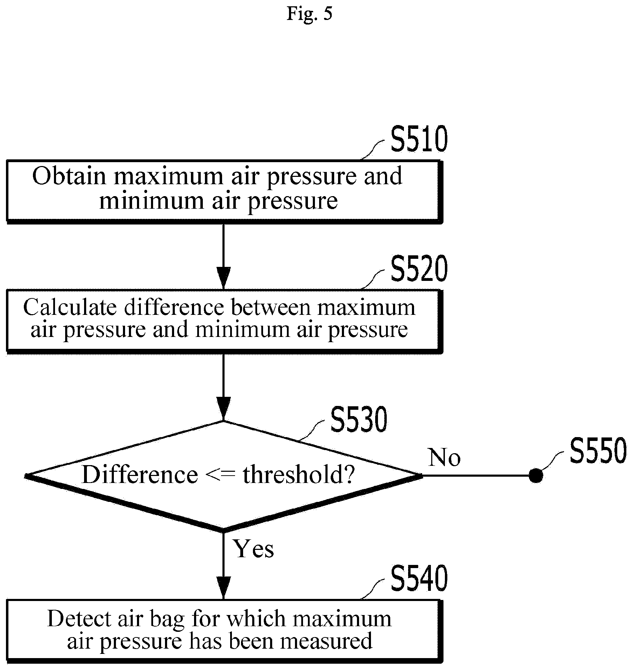

Steps S450 and S460 of Fig. 4 are described below in greater detail with reference to Fig. 5. Fig. 5 is a flowchart illustrating an external force detecting method by an external force detecting system according to an embodiment of the present invention.

In step S510, the driver 300 or processor 330 obtains the maximum air pressure and the minimum air pressure among the measured air pressures.

In step S520, the driver 300 or processor 330 may calculate the difference between the maximum air pressure and the minimum air pressure. In step S530, the driver 300 or processor 330 may compare the calculated difference with a threshold, thereby determining whether the detected air bag is pressurized by the user (particularly by the user's head).

The driver 300 or processor 330 may receive physical information about the user and calculate the threshold according to the weight of the user's head, based on the physical information.

In step S530, the driver 300 or processor 330 may compare the difference with the calculated threshold, thereby determining whether the detected air bag is pressurized by the user's head.

In step S530, the driver 300 or processor 330 may perform step S540 if the difference is equal to or larger than the threshold and, otherwise, perform step S550.

In step S540, the driver 300 or processor 330 may detect the air bag, for which the maximum air pressure is measured, and determine that the user's head is positioned on the detected air bag.

In step S550, the driver 300 or processor 330 may detect the air bag, for which the maximum air pressure is measured, and determine that a thing or the user's body portion other than the user's head is positioned on the detected air bag.

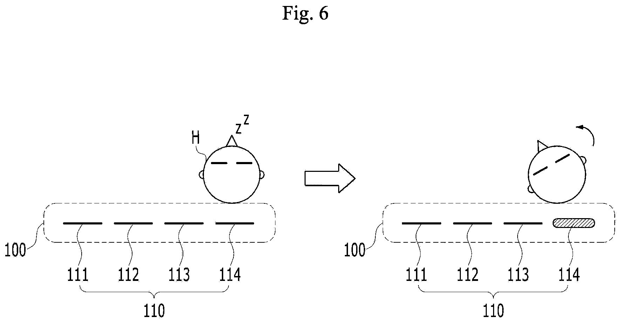

Fig. 6 is a view illustrating an example of influence on a user as an external force detecting system is driven according to an embodiment of the present invention.

Four air bags 110 are arranged side-by-side in the body 100, and the user's head H is positioned on the fourth air bag 114.

The sound receiver 340 may be disposed in the driver 300 and receive sound generated from the surroundings.

The driver 300 or processor 330 may determine whether the user is in any one abnormal state of snoring during sleep, teething during sleep, or sleep apnea based on the result of analysis of the received sound.

By the process described above in connection with Figs. 4 and 5, the driver 300 or processor 330 may detect the fourth air bag 114 to which an external force is applied.

Upon determining that the detected fourth air bag 114 is pressurized by the user and that the user is in the abnormal state, the driver 300 or processor 330 may control to inject more air than the same amount of air into the detected fourth air bag 114.

As air is injected into the fourth air bag 114, the user's head H may be moved and repositioned so that the abnormal state of the sleeping user may be mitigated or removed.

As such, the external force detecting system may use both the information about the user's abnormal state extracted and analyzed from the surrounding sound and the information about the processor where the external force is applied by the user, detected by comparing the air pressures measured on the air bags, thereby minimizing malfunctions and enabling a subsequent measure or step to move the user's head for the position of the user's head precisely grasped at the time when the user's abnormal state needs to be mitigated.

Even after performing a series of subsequent steps or measures, the external force detecting system may extract and analyze sound from the user in real0time, thereby monitoring whether the abnormal state of the sleeping user has been mitigated. Unless the abnormal state is mitigated, the external force detecting system may again detect the position of the user's head and inject air into the air bag corresponding to the newly detected position. After the user's head is repositioned, the air injected into the air bag may be fully discharged and return to its initial state.

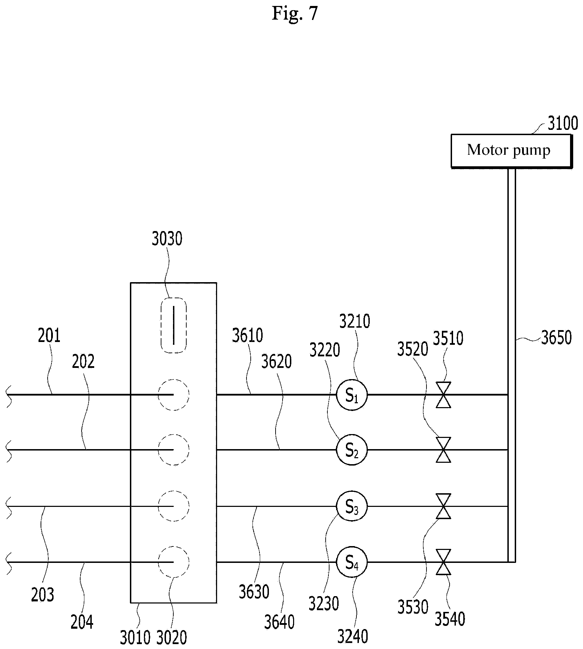

Fig. 7 is a view illustrating detailed components of a driving unit and a correction relationship therebetween, according to another embodiment of the present invention.

According to the second embodiment, a connector 3010 may be formed on one surface of the driver, and the connector 3010 may include a plurality of through holes 3020 and a power supply connecting terminal 3030 as shown in Fig. 7.

According to the second embodiment, the driver may further include a main air pathway 3650 connected with the motor pump 3100, a plurality of sub air pathways 3610, 3620, 3630, and 3640 branched from the main air pathway 3650 and respectively connected with the second ends of the air passages 201, 202, 203, and 204 in a one-to-one correspondence manner, and a valve group installed between the motor pump 3100 and the through holes 3020 to block or open the air flow.

According to the second embodiment, the air pressure sensing unit of the driver may include a plurality of air pressure sensors 3210, 3220, 3230, and 3240 respectively disposed on the sub air pathways 3610, 3620, 3630, and 3640. The valve group may include a plurality of valves 3510, 3520, 3530, and 3540 respectively disposed on the sub air pathways 3610, 3620, 3630, and 3640 between the main air pathway 3650 and the air pressure sensors 3210, 3220, 3230, and 3240.

The processor may control the operation of the individual valves of the valve group so that the same amount of air is sequentially injected into the air bags 110.

First, the processor may turn on the first valve 3510 and turn off the other valves 3520, 3530, and 3540 and then inject as much air as V into the first air bag 111. After injecting as much air as V, the processor turns off the first valve 3510, and the first air pressure sensor 3210 measures the air pressure of the first air bag 111.

Next, the processor turns on the second valve 3520 and turns off the other valves 3510, 3530, and 3540 and then inject as much air as V, which is the same amount of air injected into the first air bag 111, into the second air bag 112. After injecting as much air as V, the processor turns off the second valve 3520, and the second air pressure sensor 3220 measures the air pressure of the second air bag 112.

Next, the processor measures the air pressure of the third air bag 113 and the fourth air bag 114 in the same manner as described above.

The air injected into the air bags 110 may be discharged sequentially after the air pressure measurement is performed on the individual air bags or simultaneously after the air pressure measurement is performed on all of the air bags.

According to the second embodiment, the driver controls the operation of the motor pump 310 and the individual valves of the valve group as described above and may thus measure the air pressure of each air bag 110 under the same condition using the plurality of air pressure sensors 3210, 3220, 3230, and 3240.

While the driver according to the first embodiment measures the air pressure of all the air bags 110 using the main air pressure sensor 325, the driver according to the second embodiment measures each air bag using its respective corresponding one of the plurality of air pressure sensors 3210, 3220, 3230, and 3240.

Thus, the driver according to the first embodiment may lead to a reduction in the number of air pressure sensors which are pricey but may have a complicated driving procedure. The driver according to the second embodiment may result in a rise in cost due to the number of air pressure sensors but may have a simplified driving procedure and thus a reduced time for air pressure measurement.

However, whichever embodiment is adopted, the body and the driver are separately configured, thereby minimizing malfunctions or damage to the detailed configuration of the driver and significantly reducing the risk of electromagnetic waves. Further, it is possible to accurately detect the position of the user's head without disturbing the sleeping user and to mitigate or remove the abnormal state of the sleeping user using the detected position information about the user.

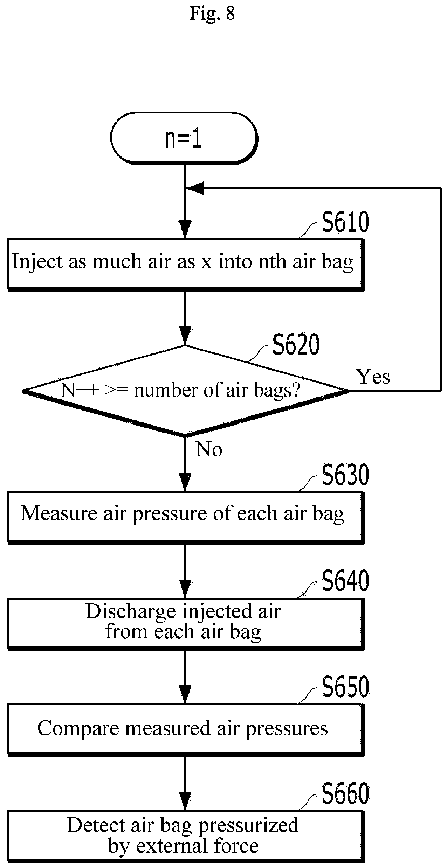

Fig. 8 is a flowchart illustrating a method for driving an external force detecting system according to another embodiment of the present invention. The driving method is described with reference to the detailed configurations of the drivers in the external force detecting system of Fig. 1 and the external force detecting system of Fig. 7.

As described above, the driver 300, which is spaced apart from, and separately configured from, the body 110, includes the air pressure sensing unit 320, the processor 330, and the motor pump 3100 which injects or discharges air via the plurality of air passages 200 individually connected with the air bags 110.

First, the driver 300 or processor 330 controls to inject the same amount of air into the plurality of same air bags 110, which stays in the initial state in the body 100 spaced apart from the driver 300. In other words, before the air is injected, the air bags 110 are in substantially the same state.

Next, the air pressure sensing unit 3210, 3220, 3230, and 3240 is spaced apart from the body 100 and measures the air pressure of each of the air bags 110 into which the same amount of air has been injected.

Specifically, in step S610, as much air as x is injected into the nth air bag by the operation of the processor 330, the motor pump 3100, and the individual valves 3510, 3520, 3530, and 3540 included in the valve group 350.

Here, n increases by one from one, and step S610 is performed repeatedly as many times as the number of the air bags.

More specifically, the driver 300 may further include a main air pathway 3650 connected with the motor pump 3100, a plurality of sub air pathways 3610, 3620, 3630, and 3640 branched from the main air pathway 3650 and connected with the air passages 200 in a one-to-one correspondence manner, and a valve group 350 installed between the motor pump 3100 and the air passages 200 to block or open the air flow.

In this case, the driver 300 or processor 330 may control the operation of the motor pump 3100 and the individual valves 3510, 3520, 3530, and 3540 included in the valve group 350 to inject as much air as x, which is the same amount of air, into the plurality of same air bags which are in the initial state or in the same state.

In step S620, if n is equal to or smaller than the number of air bags, n is increased by one, and step S610 is performed. If n is larger than the number of air bags, step S620 is performed.

In step S620, the air pressure of the nth air bag may be measured by the plurality of air pressure sensors 3210, 3220, 3230, and 3240 and, in step S630, the air injected into the nth air bag may be discharged by the operation of the processor 330, the motor pump 3100, and the individual valves 3510, 3520, 3530, and 3540 included in the valve group 350.

In step S650, the driver 300 or the processor 330 compares the measured air pressures. In step S660, the driver 300 or processor 330 detects the air bag pressurized by the external force applied to the body 100.

What has been described above in connection with Figs. 5 and 6 may be applied likewise to methods for driving an external force detecting system according to other embodiments of the present invention, and no detailed description thereof is given here.

Each step (the steps of controlling, measuring, detecting, obtaining, and determining) of the method for driving the external force detecting system described above may be performed automatically as a predetermined series of driving process when power is applied to the driver, even without the user's separate manipulation or action. The driving process may be periodically repeated.

In other words, applying power to the driver may mean a state in which driving has started or a state in which a start signal (e.g., reception of a sound) is awaited. Thus, in contrast to the typical or conventional process for driving a wired electronic product, which requires two steps (of connecting the power line and pressing the power-on button), the driving process may be simplified into one step, increasing the user convenience and satisfaction.

Some embodiments of the present invention may be implemented in the form of a computer readable recording medium that records a program to execute computer-executable commands or a program for executing at least any one of the above-described driving methods. The computer-readable storage medium may be an available medium that is accessible by a computer. The computer-readable storage medium may include a volatile medium, a non-volatile medium, a separable medium, and/or an inseparable medium. The computer-readable storage medium may include a computer storage medium. The computer storage medium may include a volatile medium, a non-volatile medium, a separable medium, and/or an inseparable medium that is implemented in any method or scheme to store computer-readable commands, data architecture, program modules, or other data or information.

All or some of the components or operations of the present invention ay be implemented in or by a computer system having a general-purpose hardware architecture or a dedicated computer, computer system, or device.

Although embodiments of the present invention have been described with reference to the accompanying drawings, It will be appreciated by one of ordinary skill in the art that the present disclosure may be implemented in other various specific forms without changing the essence or technical spirit of the present disclosure. Thus, it should be noted that the above-described embodiments are provided as examples and should not be interpreted as limiting. Each of the components may be separated into two or more units or modules to perform its function(s) or operation(s), and two or more of the components may be integrated into a single unit or module to perform their functions or operations.

It should be noted that the scope of the present invention is defined by the appended claims rather than the described description of the embodiments and include all modifications or changes made to the claims or equivalents of the claims.

## Claims

1. An external force detecting system of a pillow, comprising:
a body (100) including a plurality of same air bags (110);
a driver (300) including a motor pump (310) and a processor (330), the motor pump (310) injecting air to expand the plurality of air bags (110) or discharging air to contract the air bags(110), the processor (330) controlling operations of the motor pump (310) and detecting an air bag pressurized by an external force applied to the body (100);
a plurality of air passages (200) being identical in number to the plurality of air bags (110), the plurality of air passages (200) having first ends individually connected to the air bags (110) and second ends connected with the driver (300); and **characterised in that** the external force detecting system of a pillow further comprises
an air pressure sensing unit (320) disposed between the first ends and the motor pump (310) to measure air pressures of the air bags (110) into which the same amount of air has been injected, wherein the processor (330) obtains a maximum air pressure and a minimum air pressure among the measured air pressures, compares a difference between the maximum air pressure and the minimum air pressure with a threshold to thereby determine whether the detected air bag is pressurized by a user.

2. The external force detecting system of claim 1, wherein the processor (330) receives the user's physical information, obtains the threshold according to a weight of the user's head based on the physical information, and compares the difference with the obtained threshold to thereby determine whether the detected air bag is pressurized by the user's head.

3. The external force detecting system of claim 1, wherein the driver (300) further includes a sound receiver receiving a sound from surroundings, and wherein the processor (330) determines whether the user is in any abnormal state of snoring during sleep, teething during sleep, or sleep apnea, based on a result of analysis of the received sound.

4. The external force detecting system of claim 3, wherein when the user is in the abnormal state, the processor (330) controls to allow a larger amount of air than the same amount to be injected into the detected air bag.

5. The external force detecting system of claim 1, wherein when the driver (300) is implemented as a casing including a predetermined space, a connector (301) including a plurality of through holes (302) is formed on a first surface of the casing, wherein the second ends are coupled from an outside of the casing to the through holes (302) in a one-to-one correspondence, wherein a plurality of air pathways connected from an inside of the casing to the motor pump (310) are coupled to the second ends in a one-to-one correspondence, and wherein the air pressure sensing unit (320) is disposed in the casing.

6. The external force detecting system of claim 1, wherein the driver (300) further includes a main air pathway connected with the motor pump (310), a plurality of sub air pathways branched from the main air pathway and connected with the second ends in a one-to-one correspondence, and a valve group (350) installed between the motor pump (310) and the through holes (302) to block or open an air flow, and wherein the processor (330) controls operations of individual valves included in the valve group (350) to sequentially inject the same amount of air into the air bags.

7. The external force detecting system of claim 6, wherein the air pressure sensing unit (320) includes a plurality of air pressure sensors individually disposed on the sub air pathways, and wherein the valve group (350) includes a plurality of valves disposed between the air pressure sensors and the main air pathway.

8. The external force detecting system of claim 6, wherein the air pressure sensing unit (320) includes a main air pressure sensor disposed on the main air pathway, and wherein the valve group (350) includes a plurality of valves individually disposed on the sub air pathways and a main valve (355) disposed on the main air pathway between the main air pressure sensor and the motor pump (310).

9. The external force detecting system of claim 1, wherein the body (100) is formed of memory foam, and wherein a predetermined pattern is engraved in a surface of the body (100).

10. The external force detecting system of claim 5, wherein a power supply connecting terminal (303) separate from the through holes (302) is further included in the connector (301), and wherein when power is applied from the power supply connecting terminal (303), the driver (300) automatically initiates a predetermined series of driving processes.

11. A method for driving an external force detecting system of a pillow, according to claim 1, the method comprising:
controlling, by a driver (300), to inject the same amount of air into a plurality of same air bags (110), which are in an initial state in a body (100) spaced apart from the driver (300), via air passages individually connected with the air bags (100);
measuring, by an air pressure sensing unit (320) spaced apart from the body (100), air pressures of air bags (110) into which the same amount of air has been injected;
detecting, by the driver (300), an air bag pressurized by an external force applied to the body (100) by comparing the measured air pressures;
obtaining, by the driver (300), a maximum air pressure and a minimum air pressure among the measured air pressures; and
determining, by the driver (300), whether the detected air bag is pressurized by a user by comparing a difference between the maximum air pressure and the minimum air pressure and a threshold.

12. The method of claim 11, further comprising receiving, by the driver (300), the user's physical information and obtaining, by the driver (300), the threshold according to a weight of the user's head based on the physical information, wherein said determining includes comparing the difference with the obtained threshold to thereby determine whether the detected air bag is pressurized by the user's head.

13. The method of claim 11, further comprising:
receiving, by a sound receiver (340) disposed in the driver (300), a sound from surroundings;
determining, by the driver (300), whether the user is in any abnormal state of snoring during sleep, teething during sleep, or sleep apnea, based on a result of analysis of the received sound; and
upon determining that the detected air bag is pressurized by the user and that the user is in the abnormal state, controlling, by the driver (300), to allow a larger amount of air than the same amount to be injected into the detected air bag.

14. The method of claim 11, wherein the driver (300) further includes a motor pump (310), a main air pathway connected with the motor pump (310), a plurality of sub air pathways branched from the main air pathway and connected with the air passages (200) in a one-to-one correspondence, and a valve group (350) installed between the motor pump (310) and the air passages (200) to block or open an air flow, and wherein said controlling includes controlling operations of the motor pump (310) and individual valves included in the valve group (350) to sequentially inject the same amount of air into the plurality of same air bags (100) which are in the initial state.

15. The method of claim 11, wherein said controlling, said measuring, said detecting, said obtaining, and said determining are performed automatically as a predetermined series of driving processes when power is applied to the driver (300).

## Patentansprüche

1. System zum Detektieren einer äußeren Kraft für ein Kissen, umfassend:
einen Körper (100), der mehrere gleiche Luftkammern (110) enthält;
einen Antrieb (300), der eine Motorpumpe (310) und einen Prozessor (330) aufweist, wobei die Motorpumpe (310) Luft einbläst, um die mehreren Luftkammern (110) aufzuweiten, oder Luft ablässt, um die Luftkammern (110) zusammenzuziehen, wobei der Prozessor (330) den Betrieb der Motorpumpe (310) steuert und eine Luftkammer detektiert, die durch eine auf den Körper (100) ausgeübte äußere Kraft mit Druck beaufschlagt wird;
mehrere Luftkanäle (200), deren Anzahl mit der der Anzahl der mehreren Luftkammern (110) identisch ist, wobei die mehreren Luftkanäle (200) erste Enden aufweisen, die individuell mit den Luftkammern (110) verbunden sind, und zweite Enden aufweisen, die mit dem Antrieb (300) verbunden sind; und **dadurch gekennzeichnet, dass** das System zum Detektieren einer äußeren Kraft für ein Kissen des Weiteren umfasst:
eine Luftdruckerfassungseinheit (320), die zwischen den ersten Enden und der Motorpumpe (310) angeordnet ist, um Luftdrücke der Luftkammern (110), in die die gleiche Luftmenge eingeblasen wurde, zu messen, wobei der Prozessor (330) einen maximalen Luftdruck und einen minimalen Luftdruck unter den gemessenen Luftdrücken erhält, eine Differenz zwischen dem maximalen Luftdruck und dem minimalen Luftdruck mit einer Schwelle vergleicht, um dadurch zu bestimmen, ob die detektierte Luftkammer durch einen Benutzer mit Druck beaufschlagt ist.

2. System zum Detektieren einer äußeren Kraft nach Anspruch 1, wobei der Prozessor (330) die physischen Informationen des Benutzers empfängt, die Schwelle gemäß einem Gewicht des Kopfes des Benutzers anhand der physischen Informationen erhält und die Differenz mit der erhaltenen Schwelle vergleicht, um dadurch zu bestimmen, ob die detektierte Luftkammer durch den Kopf des Benutzers mit Druck beaufschlagt ist.

3. System zum Detektieren einer äußeren Kraft nach Anspruch 1, wobei der Antrieb (300) des Weiteren einen Geräuschempfänger umfasst, der ein Geräusch aus der Umgebung empfängt, und wobei der Prozessor (330) auf der Grundlage eines Ergebnisses der Analyse des empfangenen Geräuschs bestimmt, ob sich der Benutzer in einem anormalen Zustand des Schnarchens während des Schlafs, des Zähneknirschens während des Schlafs oder einer Schlafapnoe befindet.

4. System zum Detektieren einer äußeren Kraft nach Anspruch 3, wobei, wenn sich der Benutzer in einem anormalen Zustand befindet, der Prozessor (330) steuert, dass eine größere Luftmenge als die gleiche Menge in die detektierte Luftkammer eingeblasen werden kann.

5. System zum Detektieren einer äußeren Kraft nach Anspruch 1, wobei, wenn der Antrieb (300) als ein Gehäuse implementiert ist, das einen zuvor festgelegt Raum enthält, ein Verbinder (301), der mehrere Durchgangslöcher (302) aufweist, an einer ersten Fläche des Gehäuses gebildet ist, wobei die zweiten Enden von einer Außenseite des Gehäuses her mit den Durchgangslöchern (302) in einer Eins-zu-eins-Entsprechung gekoppelt sind, wobei mehrere Luftpfade, die von einer Innenseite des Gehäuses her mit der Motorpumpe (310) verbunden sind, mit den zweiten Enden in einer Eins-zu-eins-Entsprechung gekoppelt sind, und wobei die Luftdruckerfassungseinheit (320) in dem Gehäuse angeordnet ist.

6. System zum Detektieren einer äußeren Kraft nach Anspruch 1, wobei der Antrieb (300) des Weiteren einen Hauptluftpfad, der mit der Motorpumpe (310) verbunden ist, mehrere Nebenluftpfade, die von dem Hauptluftpfad abzweigen und mit den zweiten Enden in einer Eins-zu-Eins-Entsprechung verbunden sind, und eine Ventilgruppe (350), die zwischen der Motorpumpe (310) und den Durchgangslöchern (302) installiert ist, um einen Luftstrom zu blockieren oder zu öffnen, umfasst, und wobei der Prozessor (330) den Betrieb individueller Ventile, die in der Ventilgruppe (350) enthalten sind, steuert, um nacheinander die gleiche Luftmenge in die Luftkammern einzublasen.

7. System zum Detektieren einer äußeren Kraft nach Anspruch 6, wobei die Luftdruckerfassungseinheit (320) mehrere Luftdrucksensoren umfasst, die individuell in den Nebenluftpfaden angeordnet sind, und wobei die Ventilgruppe (350) mehrere Ventile aufweist, die zwischen den Luftdrucksensoren und dem Hauptluftpfad angeordnet sind.

8. System zum Detektieren einer äußeren Kraft nach Anspruch 6, wobei die Luftdruckerfassungseinheit (320) einen Hauptluftdrucksensor aufweist, der in dem Hauptluftpfad angeordnet ist, und wobei die Ventilgruppe (350) mehrere Ventile aufweist, die individuell in den Nebenluftpfaden angeordnet sind, und ein Hauptventil (355) aufweist, das in dem Hauptluftpfad zwischen dem Hauptluftdrucksensor und der Motorpumpe (310) angeordnet ist.

9. System zum Detektieren einer äußeren Kraft nach Anspruch 1, wobei der Körper (100) aus Formgedächtnisschaum gebildet ist, und wobei ein zuvor festgelegtes Muster in eine Fläche des Körpers (100) eingraviert ist.

10. System zum Detektieren einer äußeren Kraft nach Anspruch 5, wobei des Weiteren ein von den Durchgangslöchern (302) getrennter Stromversorgungsanschluss (303) in dem Verbinder (301) enthalten ist, und wobei, wenn Strom von dem Stromversorgungsanschluss (303) angelegt wird, der Antrieb (300) automatisch eine zuvor festgelegte Reihe von Antriebsprozessen initiiert.

11. Verfahren zum Antreiben eines Systems zum Detektieren einer äußeren Kraft für ein Kissen nach Anspruch 1, wobei das Verfahren umfasst:
Steuern, durch einen Antrieb (300), des Einblasens der gleichen Luftmenge in mehrere gleiche Luftkammern (110), die sich in einem Anfangszustand in einem von dem Antrieb (300) beabstandeten Körper (100) befinden, über mit den Luftkammern (100) individuell verbundene Luftkanäle;
Messen, durch eine von dem Körper (100) beabstandete Luftdruckerfassungseinheit (320), der Luftdrücke von Luftkammern (110), in die die gleiche Luftmenge eingeblasen wurde;
Detektieren, durch den Antrieb (300), einer Luftkammer, die durch eine auf den Körper (100) ausgeübte äußere Kraft mit Druck beaufschlagt ist, durch Vergleichen der gemessenen Luftdrücke;
Erhalten, durch den Antrieb (300), eines maximalen Luftdrucks und eines minimalen Luftdrucks unter den gemessenen Luftdrücken; und
Bestimmen, durch den Antrieb (300), ob die detektierte Luftkammer durch einen Benutzer mit Druck beaufschlagt wird, durch Vergleichen einer Differenz zwischen dem maximalen Luftdruck und dem minimalen Luftdruck und einer Schwelle.

12. Verfahren nach Anspruch 11, des Weiteren umfassend das Empfangen, durch den Antrieb (300), der physischen Informationen des Benutzers, und Erhalten, durch den Antrieb (300), der Schwelle gemäß einem Gewicht des Kopfes des Benutzers auf der Grundlage der physischen Informationen, wobei das Bestimmen das Vergleichen der Differenz mit der erhaltenen Schwelle umfasst, um dadurch zu bestimmen, ob die detektierte Luftkammer durch den Kopf des Benutzers mit Druck beaufschlagt wird.

13. Verfahren nach Anspruch 11, des Weiteren umfassend:
Empfangen, durch einen in dem Antrieb (300) angeordneten Geräuschempfänger (340), eines Geräuschs aus der Umgebung;
Bestimmen, durch den Antrieb (300), ob sich der Benutzer in einem anormalen Zustand des Schnarchens während des Schlafs, des Zähneknirschens während des Schlafs, oder der Schlafapnoe befindet, anhand eines Ergebnisses der Analyse des empfangenen Geräuschs; und
nach dem Bestimmen, dass die detektierte Luftkammer durch den Benutzer mit Druck beaufschlagt ist und dass sich der Benutzer in einem anormalen Zustand befindet, Steuern, durch den Antrieb (300), dass eine größere Luftmenge als die gleiche Menge in die detektierte Luftkammer eingeblasen werden kann.

14. Verfahren nach Anspruch 11, wobei der Antrieb (300) des Weiteren eine Motorpumpe (310), einen Hauptluftpfad, der mit der Motorpumpe (310) verbunden ist, mehrere Nebenluftpfade, die von dem Hauptluftpfad abzweigen und mit den Luftkanälen (200) in einer Eins-zu-Eins-Entsprechung verbunden sind, und eine Ventilgruppe (350), die zwischen der Motorpumpe (310) und den Durchgangslöchern (200) installiert ist, um einen Luftstrom zu blockieren oder zu öffnen, aufweist, und wobei das Steuern das Steuern von Operationen der Motorpumpe (310) und individueller Ventile, die in der Ventilgruppe (350) enthalten sind, umfasst, um nacheinander die gleiche Luftmenge in die mehreren gleichen Luftkammern (100), die sich im Ausgangszustand befinden, einzublasen.

15. Verfahren nach Anspruch 11, wobei das Steuern, das Messen, das Detektieren, das Erhalten und das Bestimmen automatisch als eine zuvor festgelegte Reihe von Antriebsprozessen durchgeführt werden, wenn dem Antrieb (300) Strom zugeführt wird.

## Revendications

1. Système de détection de force externe d'un oreiller, comprenant :
un corps (100) incluant une pluralité de mêmes coussins d'air (110) ;
un entraînement (300) incluant une motopompe (310) et un processeur (330), la motopompe (310) injectant de l'air pour déployer la pluralité de coussins d'air (110) ou évacuant de l'air pour contracter les coussins d'air (110), le processeur (330) commandant des manœuvres de la motopompe (310) et détectant un coussin d'air mis sous pression par une force externe appliquée au corps (100) ;
une pluralité de passages d'air (200) étant identique en nombre à la pluralité de coussins d'air (110), la pluralité de passages d'air (200) ayant des premières extrémités individuellement reliées aux coussins d'air (110) et des secondes extrémités reliées à l'entraînement (300) ; et **caractérisé en ce que** le système de détection de force externe d'un oreiller comprend en outre :
une unité de détection de pression d'air (320) disposée entre les premières extrémités et la motopompe (310) pour mesurer des pressions d'air des coussins d'air (110) dans lesquels la même quantité d'air a été injectée, dans lequel le processeur (330) obtient une pression d'air maximale et une pression d'air minimale parmi les pressions d'air mesurées, compare une différence entre la pression d'air maximale et la pression d'air minimale avec un seuil pour ainsi déterminer si le coussin d'air détecté est mis sous pression par un utilisateur.

2. Système de détection de force externe selon la revendication 1, dans lequel le processeur (330) reçoit les informations physiques de l'utilisateur, obtient le seuil en fonction d'un poids de la tête de l'utilisateur sur la base des informations physiques, et compare la différence avec le seuil obtenu pour ainsi déterminer si le coussin d'air détecté est mis sous pression par la tête de l'utilisateur.

3. Système de détection de force externe selon la revendication 1, dans lequel l'entraînement (300) inclut en outre un récepteur de son recevant un son des alentours, et dans lequel le processeur (330) détermine si l'utilisateur est dans un état anormal quelconque parmi un ronflement pendant le sommeil, une poussée de dents pendant le sommeil ou une apnée du sommeil, sur la base d'un résultat d'analyse du son reçu.

4. Système de détection de force externe selon la revendication 3, dans lequel, lorsque l'utilisateur est dans l'état anormal, le processeur (330) commande d'admettre une plus grande quantité d'air que la même quantité à injecter dans le coussin d'air détecté.

5. Système de détection de force externe selon la revendication 1, dans lequel, lorsque l'entraînement (300) est mis en œuvre sous la forme d'un boîtier incluant un espace prédéterminé, un connecteur (301) incluant une pluralité de trous traversants (302) est formé sur une première surface du boîtier, dans lequel les secondes extrémités sont couplées depuis un extérieur du boîtier aux trous traversants (302) dans une correspondance un à un, dans lequel une pluralité de trajets d'air reliés depuis un intérieur du boîtier à la motopompe (310) sont couplés aux secondes extrémités dans une correspondance un à un, et dans lequel l'unité de détection de pression d'air (320) est disposée dans le boîtier.

6. Système de détection de force externe selon la revendication 1, dans lequel l'entraînement (300) inclut en outre un trajet d'air principal relié à la motopompe (310), une pluralité de trajets d'air secondaires bifurquant à partir du trajet d'air principal et reliés aux secondes extrémités dans une correspondance un à un, et un groupe de soupapes (350) installé entre la motopompe (310) et les trous traversants (302) pour bloquer ou ouvrir un écoulement d'air, et dans lequel le processeur (330) commande des manœuvres de soupapes individuelles incluses dans le groupe de soupapes (50) pour injecter séquentiellement la même quantité d'air dans les coussins d'air.

7. Système de détection de force externe selon la revendication 6, dans lequel l'unité de détection de pression d'air (320) inclut une pluralité de capteurs de pression d'air disposés individuellement sur les trajets d'air secondaires, et dans lequel le groupe de soupapes (350) inclut une pluralité de soupapes disposées entre les capteurs de pression d'air et le trajet d'air principal.

8. Système de détection de force externe selon la revendication 6, dans lequel l'unité de détection de pression d'air (320) inclut un capteur de pression d'air principal disposé sur le trajet d'air principal, et dans lequel le groupe de soupapes (350) inclut une pluralité de soupapes disposées individuellement sur les trajets d'air secondaires et une soupape principale (355) disposée sur le trajet d'air principal entre le capteur de pression d'air principal et la motopompe (310).

9. Système de détection de force externe selon la revendication 1, dans lequel le corps (100) est formé d'une mousse à mémoire de forme, et dans lequel un motif prédéterminé est gravé dans une surface du corps (100).

10. Système de détection de force externe selon la revendication 5, dans lequel une borne de connexion d'alimentation en courant (303) séparée des trous traversants (302) est en outre incluse dans le connecteur (301), et dans lequel, lorsque du courant est appliqué à partir de la borne de connexion d'alimentation en courant (303), l'entraînement (300) initie automatiquement une série prédéterminée de processus de pilotage.

11. Procédé pour piloter un système de détection de force externe d'un oreiller selon la revendication 1, le procédé comprenant de :
commander, par un entraînement (300), d'injecter la même quantité d'air dans une pluralité de mêmes coussins d'air (110), qui sont dans un état initial dans un corps (100) espacé de l'entraînement (300), via des passages d'air individuellement reliés aux coussins d'air (100) ;
mesurer, par une unité de détection de pression d'air (320) espacée du corps (100), des pressions d'air de coussins d'air (110) dans lesquels la même quantité d'air a été injectée ;
détecter, par l'entraînement (300), un coussin d'air mis sous pression par une force externe appliquée au corps (100) en comparant les pressions d'air mesurées ;
obtenir, par l'entraînement (300), une pression d'air maximale et une pression d'air minimale parmi les pressions d'air mesurées ; et
déterminer, par l'entraînement (300), si le coussin d'air détecté est mis sous pression par un utilisateur en comparant une différence entre la pression d'air maximale et la pression d'air minimale et un seuil.

12. Procédé selon la revendication 11, comprenant en outre de recevoir, par l'entraînement (300), les informations physiques de l'utilisateur et d'obtenir, par l'entraînement (300), le seuil en fonction d'un poids de la tête de l'utilisateur sur la base des informations physiques, dans lequel ladite détermination inclut de comparer la différence avec le seuil obtenu pour ainsi déterminer si le coussin d'air détecté est mis sous pression par la tête de l'utilisateur.

13. Procédé selon la revendication 11, comprenant en outre de :
recevoir, par un récepteur de son (340) disposé dans l'entraînement (300), un son des alentours ;
déterminer, par l'entraînement (300), si l'utilisateur est dans un état anormal quelconque parmi un ronflement pendant le sommeil, une poussée de dents pendant le sommeil ou une apnée du sommeil, sur la base d'un résultat d'analyse du son reçu ; et
lorsqu'il est déterminé que le coussin d'air détecté est mis sous pression par l'utilisateur et que l'utilisateur est dans un état anormal, commander, par l'entraînement (300), d'admettre une plus grande quantité d'air que la même quantité à injecter dans le coussin d'air détecté.

14. Procédé selon la revendication 11, dans lequel l'entraînement (300) inclut en outre une motopompe (310), un trajet d'air principal relié à la motopompe (310), une pluralité de trajets d'air secondaires bifurquant à partir du trajet d'air principal et reliés aux passages d'air (200) dans une correspondance un à un, et un groupe de soupapes (350) installé entre la motopompe (310) et les passages d'air (200) pour bloquer ou ouvrir un écoulement d'air, et dans lequel ladite commande inclut de commander des manœuvres de la motopompe (310) et de soupapes individuelles incluses dans le groupe de soupapes (350) pour injecter séquentiellement la même quantité d'air dans la pluralité de mêmes coussins d'air (100) qui sont à l'état initial.

15. Procédé selon la revendication 11, dans lequel ladite commande, ladite mesure, ladite détection, ladite obtention et ladite détermination sont réalisées automatiquement comme une série prédéterminée de processus d'entraînement lorsque du courant est amené à l'entraînement (300).
